(19) Europäisches Patentamt
European Patent Office
Office européen des brevets

(11) Publication number: **0 028 126**
**B1**

(12) **EUROPEAN PATENT SPECIFICATION**

(45) Date of publication of patent specification: **07.03.84**

(51) Int. Cl.³: **C 08 B 37/08**, B 01 D 13/04

(21) Application number: **80303739.9**

(22) Date of filing: **22.10.80**

(54) **Material produced from cross-linked ionic chitin derivative, its preparation and use.**

(30) Priority: **24.10.79 JP 137337/79**

(43) Date of publication of application:
**06.05.81 Bulletin 81/18**

(45) Publication of the grant of the patent:
**07.03.84 Bulletin 84/10**

(84) Designated Contracting States:
**DE FR GB NL SE**

(56) References cited:
EP - A - 0 013 181
DE - A - 894 993
US - A - 4 111 810

(73) Proprietor: **KUREHA KAGAKU KOGYO KABUSHIKI KAISHA**
**9-11 Horidome-cho 1-chome Nihonbashi Chuo-ku**
**Tokyo (JP)**

(72) Inventor: **Koshugi, Junichi**
**No. 22 Daiichi-Kurehasoh 3-10-13 Nerima**
**Nerima-ku**
**Tokyo (JP)**

(74) Representative: **Myerscough, Philip Boyd et al,**
**J.A.Kemp & Co. 14, South Square Gray's Inn**
**London, WC1R 5EU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 028 126 B1

# Material produced from cross-linked ionic chitin derivative, its preparation and use

The present invention concerns a material produced from a cross-linked, ionic, chitin derivative, to a process for the preparation of this material and to its use.

Fiber-forming polysaccharides occurring in nature can be roughly divided into collagen in higher animals, chitin in arthropods and lower animals and cellulose in higher plants. The frames of living things are made from the products formed by sedimentation of apatite, calcium carbonate, lignin, etc onto these polysaccharides respectively.

Chitin is a high polymeric substance comprising 1,4-beta bonded N-acetyl-D-glucosamine. The amount of chitin in nature compares favorably to that of cellulose. However, since chitin has an extremely high crystallinity and the inter-molecular bonding between chitin molecules by the amino-acetyl groups is very strong and stable, an economical solvent which favorably dissolves, disperses or swells chitin as an alkali does for cellulose has not yet been found.

A method for producing a water-soluble derivative of chitin is proposed in EP—A—0 013 512. A material derived from this water-soluble derivative of chitin is disclosed in EP—A—0 013 181 (published 9th July, 1980). EP—A—0 013 181 describes the possibility of obtaining various shaped materials having amphoteric ion-exchangability. In EP—A—0 013 181, chitin derivatives obtained by acylation of a de-N-acetylated and etherified chitin may subsequently be cross-linked. The acylation may be conducted so that a material having at least an outer surface of acylated de-N-acetylated and etherified chitin is produced.

However, in addition to the problem concerning the inability of the shaped material to adsorb some high molecular weight substances, an improvement of the mechanical strength of such materials has been desired, particularly for certain fields of use. EP—A—0 018 131 (published 29th October, 1980) discloses a solution to this problem by cross-linking chitin, carboxyalkylating the cross-linked chitin, and then de-N-acetylating the carboxyalkylated cross-linked chitin. The amphoteric ion-exchanger thus obtained is excellent in adsorbing various substances of high moleuclar weight.

However, when the amphoteric ion-exchanger is used as an adsorbent in blood dialysis, the ion-exchanger also adsorbs high molecular weight substances, such as proteins, which should not be removed together with the toxic substances which should be removed. As a result the ability of the ion-exchanger to remove the toxic substances is reduced. Similar results appear where the ion-exchanger is used as a filler in a chromatograph column. The ability of the column to separate substances gradually decreases.

It has now been found that these problems can be overcome by acylating or treating with acid the ion-exchanger to substantially eliminate or reduce the ionic groups from its surface.

Accordingly, the present invention provides a material produced by acylating or treating with an acid a surface of a material composed of a cross-linked ionic chitin derivative comprising a structural unit represented by the formula:

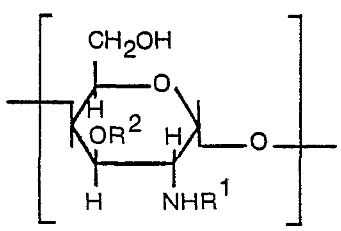

or

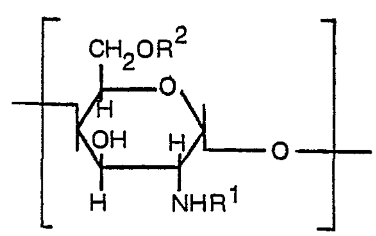

wherein

$R^1$ denotes a hydrogen atom, a group of formula $-C_nH_{2n+1}$, a group of formula $-(CH_2)_nOH$ or a group of formula $-CO(CH_2)_{n-1}CH_3$ and $R^2$ denotes a hydrogen atom, a group of formula $-C_{n'}H_{2n'+1}$, a group of formula $-(CH_2)_{n'}OH$, a group of formula

$$-(CH_2)_{n'}-\underset{\displaystyle OH}{\underset{|}{C}}H.CH_2OH$$

or a group of formula $-(CH_2)_{n'}COOM$, wherein *n* is an integer of 1 to 20, *n'* is an integer of 1 to 3, and M is a hydrogen atom, an alkali metal or an atomic equivalent amount of an alkaline earth metal.

Such a material can be produced by partially treating a cross-linked ionic chitin derivative with an organic acid anhydride or a mixture thereof with an organic acid to effect an acylation or with an aqueous mineral acid solution to effect an acid-treatment.

The present invention therefore also provides a process for the preparation of a material of the invention, which process comprises:

(i) cross-linking natural chitin or regenerated chitin with a cross-linking agent,

(ii) reacting the cross-linked chitin with an etherifying agent to introduce the $OR^2$ group at the 3-or 6- position of the pyranose ring of structural units of the cross-linked chitin, wherein $R^2$ is as defined in above,

(iii) de-acetylating the cross-linked etherified chitin, and

(iv) acylating or treating with acid the surface of the de-acetylated, cross-linked, etherified chitin.

The acylation or treatment with acid can cause the formation of one or more of a chitin, N-acylated carboxyalkylchitosan of formula (VII): $-\!\!\!\!+\!C_6H_8O_3(OCH_2COOH)NHCOR\!+\!\!\!\!-$ wherein R represents $-(CH_2)_mCH_3$ and *m* is 0 or an integer from 1 to 20, N-acylated chitosan and N-, O-diacylated chitosan on at least the surface of the material.

In the description which follows, reference will be made to the accompanying drawing in which the sole Figure shows the results of Example 6 below of testing the mechanical strength of material according to the invention (A) and comparative material (B).

The material of the present invention has a surface layer comprising an acylated product and/or a deacetylated product as mentioned hereinafter. Its interior comprises the cross-linked substance. The material of the present invention can exhibit excellent mechanical strength. Consequently it can be employed as material for capsules, for dialysis and for an ion-exchanger. It may be used in a wide range of applications such as a material for biofunctions, for collecting heavy metals, as a base material on which an enzyme, antigen or antibody is to be immobilised and for ultrafiltration. In addition, in the course of producing the material of the present invention, since agglomeration, such as that experienced in respect of previous amphoteric ion-exchanger bodies because of the functional groups on their surfaces, does not occur during de-salting, the material can be very easily handled.

The cross-linked ionic chitin derivative that is acylated or treated with an acid has structural units represented by the formula (I):

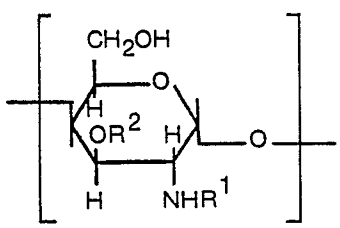

or

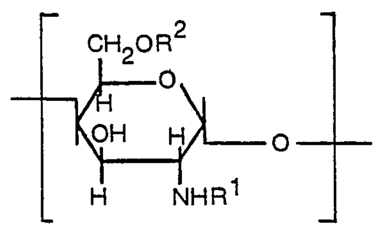

(I)

wherein

$R^1$ denotes a hydrogen atom, an alkyl group of 1 to 20 carbon atoms, an omega-hydroxyalkyl group of 1 to 20 carbon atoms or an acyl group of 2 to 21 carbon atoms and $R^2$ denotes a hydrogen atom, an alkyl group of 1 to 3 carbon atoms, an omega-hydroxyalkyl group of 1 to 3 carbon atoms, a group represented by

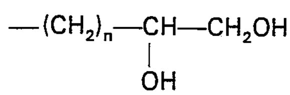

wherein

*n* is 1 to 3, a carboxyalkyl group of 2 to 4 carbon atoms or a carboxyalkyl group of 2 to 4 carbon atoms wherein the hydrogen atom of the carboxyl group is replaced by an alkali metal or an atomic equivalent of an alkaline earth metal. Preferably these are the main structural units of the cross-linked ionic chitin derivative.

The cross-linked ionic chitin derivative may also contain structural units of formula (I-a):

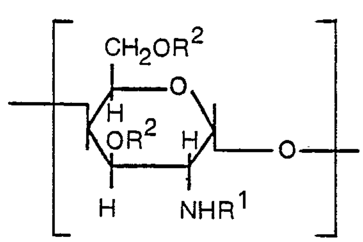

(I–a)

wherein

$R^1$ and $R^2$ are as defined above.

The ionic chitin derivative, for instance, includes carboxymethylchitin, carboxymethylchitosan, ethylchitosan, glycol chitosan, glyceride chitosan, N-monoethylglycol chitosan, N-monoethylcarboxymethyl chitosan, N-propylcarboxymethylchitosan, N-propyllauryl-carboxyethylchitosan, etc. and is cross-linked at the 3 and 6′ positions or 3, N and 6′ positions. Cross-linking at the 3 and 6′ positions is illustrated below as Type I. Cross-linking at the 3, N and 6′ positions is illustrated below as Type II.

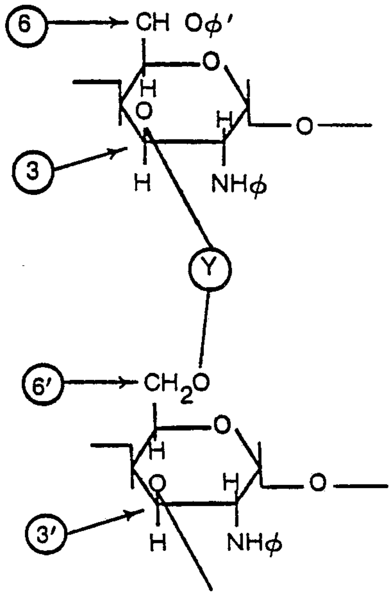

Type I

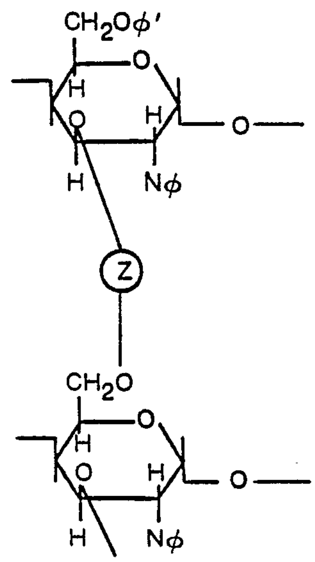

Type II

wherein

Y and Z are each cross-linking groups and $\phi$ and $\phi'$ are each functional groups. The state of cross-linking is decided by the kind of the cross-linking agent. Either di- or tri-functional agents may be used. The degree of cross-linking is not specifically restricted. However, it is usually from 0.01 to 0.3 per pyranose unit.

A cross-linked product of Type I is obtainable, for instance, by the following method of production: Chitin is used as a raw material. Chitin has the formula

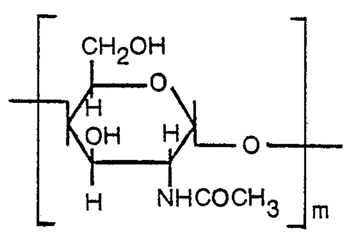

wherein

m is an integer. After pulverizing the chitin into a suitable size (in the case of using regenerated chitin, the shape may be optionally decided), the particles of chitin are immersed in an aqueous alkaline solution at a high alkali concentration to swell the intermicellular space. In this case, an aqueous alkaline solution of a normality of 10 to 15 of sodium, potassium or lithium hydroxide is used in an amount 3 to 10 times, preferably 3 to 5 times, by weight of the chitin starting material. Immersion can be effected at a temperature of 5 to 20°C.

Next, in order to make the cross-linking of the chitin easier, the alkali-immersed chitin is subjected to compression or freezing to remove excess aqueous alkaline solution. Freezing particularly aids cross-linking because the chitin micelles are opened by the freezing and alkali is retained in the minute interspaces between chitin molecules. The conditions of the freeze-treatment are, depending on the desired degree of opening chitin micelle, at a temperature of −3° to −30°C, preferably −10 to −20°C, for from 3 to 24 hours.

The cross-linking reaction can be carried out by adding a cross-linking agent, represented by the general formulae (II) to (V):

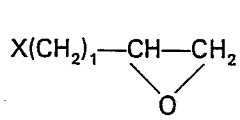 (II)

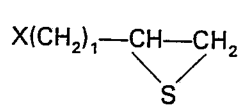 (III),

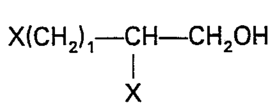 (IV) or

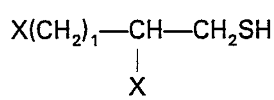 (V)

wherein

X denotes a halogen atom and 1 is an integer of 1, 2 or 3, in an amount of from 0.1 to 3 times, preferably 0.5 to 2 times, by weight of the chitin starting material to the chitin which has been freeze-treated or from which excess alkali has been removed by compression. This step is effected at a temperature lower than room temperature, preferably at a subzero temperature. This low temperature is maintained for 5 to 48 hours.

An example of the cross-linked chitin obtainable by this reaction is shown below in general formula (VI):

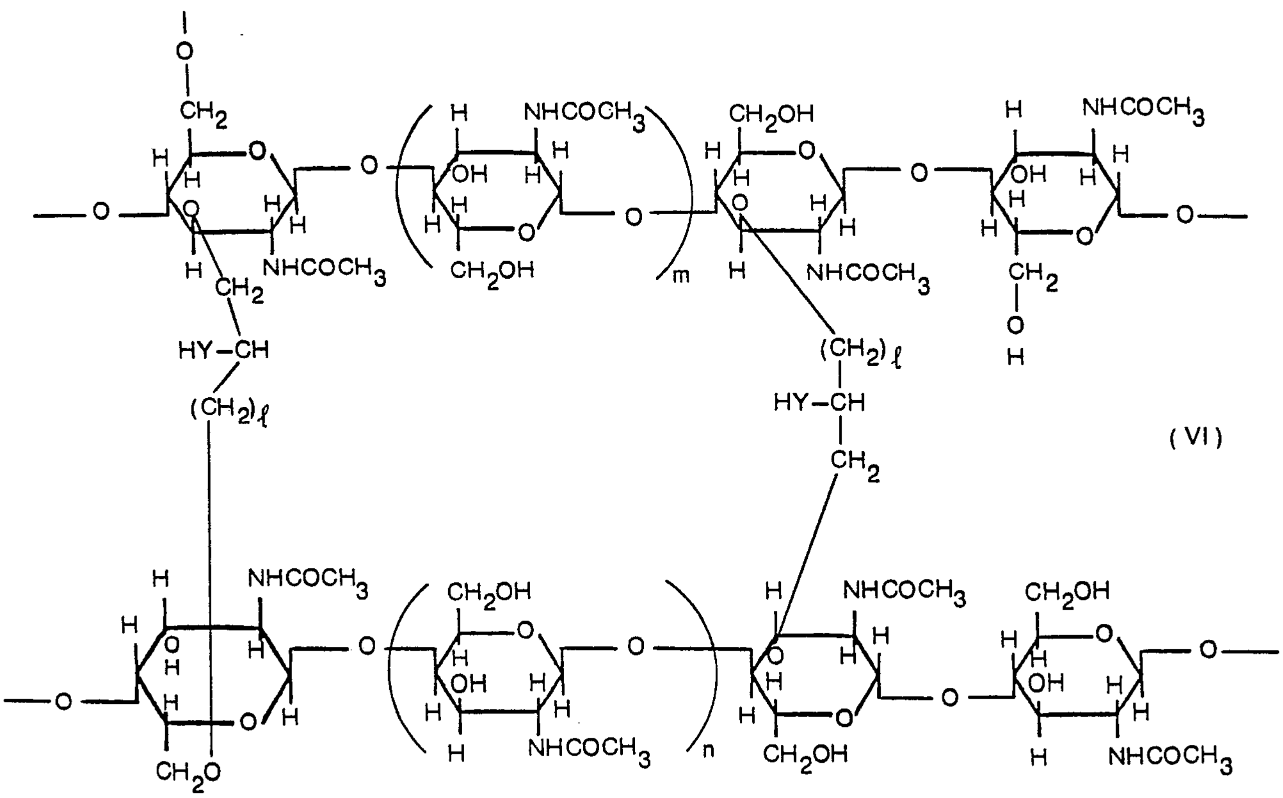

(VI)

wherein l, m and n are each integers and Y is oxygen atom or sulfur atom.

As is clearly seen in formula (VI) cross-linking is carried out via hydroxyl group(s) or hydroxymethyl group(s) in the same manner as for the carboxyalkylation, etc. shown later. Accordingly, the degree of cross-linking is decided with regard to the degree of carboxyalkylation. In the present invention, the degree of cross-linking is preferably 0.01 to 0.3 per N-acetyl-D-glucosamine unit, the degree of cross-linking being obtained from elementary analytical data of the cross-linked product.

Cross-linking agents represented by the formulae (II) to (V) include epichlorohydrin, 1-bromo-3,4-epoxybutane, 1-bromo-4,5-epoxypentane, 1-chloro-2,3-epithiopropane, 1-bromo-2,3-epithiopropane, 1-bromo-3,4-epithiobutane, 1-bromo-4,5-epithiopentane, 2,3-dibromopropanol, 2,4-dibromobutanol, 2,5-dibromopentanol, 2,3-dibromopropanethiol, 2,4-dibromobutanethiol, and 2,5-dibromopentane-thiol. Epichlorohydrin, 2,3-dibromopropanol and 1-chloro-2,3-epithiopropane are preferred. Cross-linking agents for the cross-linked state Type II include cyanuric chloride and cyanuric bromide.

Next, the $R^2$ substituent can be introduced into the cross-linked chitin. This may be achieved by reacting the cross-linked chitin with the appropriate etherifying agent. The resulting product can have a degree of $R^2$ substitution of 0.1 to 0.9, preferably 0.3 to 0.9, per N-acetyl glucosamine unit. The product is heated in an aqueous alkaline solution of sodium hydroxide or potassium hydroxide. For instance, cross-linked carboxyalkyl-chitin can be prepared as follows:

The alkali-containing cross-linked chitin described above is dispersed in an organic solvent containing an etherifying agent at a temperature of 0 to 30° for 1 to 72 hours, preferably 5 to 12 hours, to cause reaction. In this case, because of the evolution of heat due to neutralization, the temperature is preferably kept at first at 0 to 10°C and then the reaction is carried at a predetermined temperature. After the reaction is over, the solvent is distilled off. The residue is dispersed in water. The dispersion is neutralized. The neutralizate is filtered to collect the reaction product. The product is de-salted and dried to obtain a cross-linked carboxyalkylchitin. The organic solvent used in the carboxyalkylation is

preferably selected from methanol, ethanol, propyl alcohol, isopropyl alcohol, acetone and dimethylformamide.

The etherifying agent used in the carboxyalkylation is a compound represented by the formula:

$$X(CH_2)_n—COOH$$

wherein

X denotes an atom of chlorine or bromine and n is an integer of 1, 2 or 3. Such etherifying agents include monochloroacetic acid, monobromoacetic acid, beta-monochloropropionic acid, beta-monobromopropionic acid, gamma-monochlorobutyric acid, gamma-monobromobutyric acid. The etherifying agent is generally an amount of 1 to 3 times the molecular weight of the chitin starting material. A water-insoluble derivative of chitin, such as cross-linked carboxymethylchitin and cross-linked carboxypropylchitin, can be obtained. The degree of carboxyalkylation is generally of 0.1 to 0.9, preferably 0.3 to 0.9. The degree of carboxyalkylation is obtained by elementary analysis of the product and by titration of the carboxyalkylated product, after its conversion to a salt having a pK of 4.3, against an aqueous hydrochloric acid solution of a specified normality.

The material according to the present invention is derived from a de-acetylated product of the cross-linked $R^2$-substituted chitin. For example, a cross-linked carboxyalkylchitin is treated with an aqueous alkaline solution containing a high concentration of alkali, preferably of a normality of 4 to 15, at 65 to 150°C, preferably 65 to 110°C, for 0.1 to 48 hours to effect de-acetylation. The cross-linked carboxyalkylchitin used as a starting material of this de-acetylation may be the solvent-including product of the carboxyalkylation which has been effected in an organic solvent obtained by filtering and washing with ethanol and acetone, a dried product or a product containing water. When the cross-linked carboxyalkyl chitin contains water the normality of alkali for the de-acetylation should be corrected to the above mentioned range. In the case where the de-acetylation temperature is lower than the designated one, it takes much longer to complete the reaction. On the other hand, where the temperature is higher, there is a reduction in the molecular weight of the product.

Since the carboxyalkyl group is bonded via the ether linkage, it is not removed in the de-acetylation, even when heated in the aqueous alkaline solution.

The degree of de-acetylation is obtained by elementary analysis of the product and is generally 0.1 to 1, preferably 0.3 to 1.

According to the present invention, the deionization of the surface of the cross-lined ionic chitin derivative represented by the formula (I) may be carried out in a simple manner. For example, by bringing a gel of the ionic cross-linked derivative into contact with a solution of an organic acid anhydride or a mixture of the anhydride and the organic acid or with a vapour of the anhydride, deionization can be effected by acylation in a very short period of time to obtain a material of the invention. The mechanism of this reaction has not yet been elucidated. However, reaction occurs instantaneously from the surface of the gel of the substance of formula (I) after contact with the organic acid anhydride. The reaction product of the deionization includes a chitin, a N-acylated carboxyalkylchitosan of formula (VII), a de-carboxyalkylated and N-acylated chitosan of formula (VIII) and a N-, O-acylated chitosan of formula (IX):

$$-[C_6H_8O_3(OCH_2COOH)NHCOR]- \quad (VII)$$

$$-[C_6H_8O_3(OH)NHCOR]- \quad (VIII)$$

$$-[C_6H_8O_3(OCOR)NHCOR]- \quad (IX)$$

wherein

R represents $—CH_2)_mCH_3$ and *m* is 0 or an integer of 1 to 20. However, the proportion of structural units represented by the formula (VII) occupying the surface of the material according to the invention is almost zero. Instead, structural units represented by the formulae (VIII) and (IX), particularly those represented by the formula (IX), occupy the larger part of the surface. On the other hand, in the interior of the shaped material the unreacted ionic cross-linked derivative of chitin or its salt remains as the structural units. This, of course, depends on the degree of the acylation deionisation reaction. Thus in one embodiment the material according to the present invention is the product of the treatment of the surface of a cross-linked ionic derivative of chitin by acylation.

The organic acid and acid anhydrides which can be employed as the acylating agent are preferably acetic acid, propionic acid, butyric acid and valeric acid, and their acid anhydrides. However, the carbon chain of the organic acid or its anhydride may be longer, for example up to 21 carbon atoms. These acids and acid anhydrides respectively may be used singly or as a mixture of more than one. They may be employed without dilution or after dilution with an organic solvent which does not react with the organic acid or anhydride. The temperature of acylation is preferably 5 to 80°C, more preferably 5 to 60°C. In the case where the organic acid anhydride is used in gaseous form, the temperature may be still higher.

The state in which the gel of the ionic crosslinked derivative of chitin and the organic acid anhydride contact one another can be suitably selected according to the intended usage and the desired form of the material of the present invention. For instance, spherical particles of the gel, which contain water, are dispersed under agitation in the organic acid anhydride. In this state, acylation occurs instantly from the surface of the particles to form a membrane of the acylated product. In the case where the reaction is allowed to proceed further, the organic acid anhydride diffuses into the inner part of the particles and forms a deionized layer of the desired thicknesses. After the reaction had proceeded for a predetermined period of time, the product was separated from the reaction mixture and washed with water to remove unreacted organic acid anhydride. Spherical bodies of the material of the present invention were obtained. The thickness and packing density of the acylated membrane is controllable by changing the concentration and amount of the acylating agent, the reaction period and the reaction temperature. Usually the thickness is from $10^{-7}$ to $10^{-3}$m (0.1 to 1,000 microns) and the packing density corresponds to a limit of 500 to 400,000 on the molecular weight of molecules which can pass through the membrane.

In the case where the ionic cross-linked derivative of chitin is cationic or amphoteric, the material of the present invention can be obtained by treatment with, as shown above, the acylating agent. On the other hand, in the case where the ionic cross-linked derivative of chitin is anionic, the material of the present invention may be obtained by treatment with an aqueous dilute acidic solution, such as hydrochloric acid or sulfuric acid. The acid treatment eliminates the anionic carboxyalkyl group of carboxyalkyl derivatives by hydrolysis. Of course, the acid treatment may be carried out with the acylating agent.

The product of the present invention may take any shape depending on the shape of the substance of formula (I). That substance may be spherical, fibrous, a film or hollow as disclosed in EP—A—0 013 181.

Since the material of the present invention is made up of the cross-linked product of an ionic derivative of chitin and of the acylated product of the cross-linked product, which is permeable to substances, chemically stable and possesses bio-compatibility, the material can be applied in a wide range of uses. For example, the material of the invention can be used in the purification and separation of a glycoprotein. Only a glycoprotein of a molecular weight smaller than a defined value can be dispersed into the inner part of hollow particles of the material of the present invention when such a material is put into a solution of a mixture of glycoproteins of various molecular weights. Further, it is possible to use material of the present invention in the form of hollow fibers or of films in the same manner as conventional material for dialysis.

Since proteins are not so much adsorbed on the surface of the material of the present invention, it is possible to use the material as an ion-exchanger while taking advantage of the small reduction of the adsorption velocity in use, the controllability of the permeability to substances and the ionic groups of the material of the present invention. In addition, since the material of the present invention is stable and safe in living bodies, it is possible to use it in various applications to do with living bodies, for instance in blood perfusion, as an adsorbent for internal administration to adsorb and remove gastrointestinal toxins or as a covering material of adsorbents.

The following Examples illustrate the present invention.

## Example 1

After neutralizing and de-salting by washing with water, a gel of the sodium salt of cross-linked chitin derivative in the shape of spherical particles of $5 \times 10^{-5}$ to $1.5 \times 10^{-4}$m (50 to 150 microns) in diameter with a degree of cross-linking with epichlorohydrin of 0.1, a degree of carboxymethylation of 0.7 and a degree of deacetylation of 0.9, was separated by filtration. The particles of the gel had agglomerated because of the de-salting treatment.

$2 \times 10^{-1}$ Kg (200 g) [dried weight: $10^{-2}$ kg (10 g)] of the starting material, the gel, were dispersed into $2 \times 10^{-3}m^3$ (2 liters) of a 1:1 by volume mixture of toluene and acetic anhydride in a flask.

The contents of the flask were agitated for 5 min at a room temperature for acylation to occur. After the reaction was over, the gel was separated by filtration, washed with ethanol, and separately neutralized in another flask containing $2 \times 10^{-3}m^3$ (2 liters) of distilled water.

After neutralization, the product was separated again by filtration, washed with distilled water and subjected to de-salting to obtain a shaped material of the present invention. The size of the spherical particles of the material was the same as that of the starting material. However, interparticular agglomeration by de-salting had not occurred. After drying, the material showed two infrared absorption peaks, respectively at 1680 and 1500 $cm^{-1}$. This fact confirmed the presence of carboxymethyl groups and amino groups. Results of the determination and comparison of the ion-exchange capacity of the product and the starting material are shown in Table 1. As is seen in Table 1, the product had the same ion-exchange capacity as the starting material. When the starting material was treated in an aqueous 2N hydrochloric acid solution for 5 hours at 50°C, it dissolved completely. However, when the product was treated in the same manner it retained its original shape. The insoluble

matter to the above-mentioned hydrochloric acid solution was one percent by weight of the dried gel and it was identified as chitin from i.r. spectroscopy and the elementary analysis.

TABLE 1
Comparison of Ion-Exchange Capacity

| Specimen | Cation-Exchange Capacity (meq/g) (eq/kg) | Anion-Exchange Capacity (meq/g) (eq/kg) |
|---|---|---|
| Starting material | 3.8 | 5.0 |
| Product of Example 1 | 3.7 | 4.9 |

Example 2

$10^{-2}$Kg (ten grams) of, as starting material, fibers of a cross-linked chitin derivative 2 × $10^{-4}$m (200 microns) in diameter with a degree of cross-linking by cyanuric chloride of 0.1 and a degree of deacetylation of 0.9 were swelled in water. The thus obtained fibrous gel was immersed in $10^{-3}m^3$ (one liter) of propionic anhydride at 40°C for 10 min. The thus treated fibers were separated, washed with ethanol and dried. The starting material and the treated fibers were cut into $10^{-2}$m (10 mm) lengths. Specimens were treated in 5 × $10^{-4}m^3$ (500 ml) of an aqueous 2N hydrochloric acid solution at 50°C for 5 hours. While the starting material completely dissolved, the treated fibers left an insoluble substance. After separating the insoluble substance by filtration, it was washed with water and its cross-section was examined under a microscope. It was discovered that the insoluble substance was a hollow fiber. From this result, it was considered that only the surface of the starting material was insolubilized.

Since the insoluble substance did not show an infrared absorption at 1500 to 1530 $cm^{-1}$ attributable to an amino group, but instead showed infrared absorptions near 1650 and 1550 $cm^{-1}$, respectively, the presence of amide group was presumed. From elementary analysis, the insoluble substance was identified as N-propionylchitosan.

When the solutions obtained by immersing the starting material and the product (treated with propionic anhydride) in the hydrochloric acid solution were neutralized with an aqueous 2N sodium hydroxide solution, white gel-like substances were obtained. After treating these gel-like substances by de-salting, they were dried and examined by infrared spectroscopy and elementary analysis. Both gel-like substances showed an infrared absorption at 1500 to 1530 $cm^{-1}$ attributable to an amino group. From this and their elementary analytical data, they were identified as chitosan. For confirmation, the hydrochloric acid solutions of the starting material and of the product were brought into reaction with iodine in the presence of sulfric acid. A purplish coloration was obtained in both solutions.

Example 3

$10^{-2}$Kg (10 g) of spherical particles of a cross-linked chitin derivative with a degree of cross-linking with epichlorohydrin of 0.2, a degree of hydroxyethylation of 0.7 and a degree of deacetylation of 0.9, and $10^{-2}$ kg (10 g) of a gel-like cross-linked chitin derivative with a degree of cross-linking with epichlorohydrin of 0.1, a degree of ethylation of 0.5 and a degree of deacetylation of 0.8 were respectively swollen in water. After removing excess water, both swollen gels were respectively dispersed in $10^{-3}m^3$ (one liter) of a 1:3 by volume mixture of capric anhydride and benzene at 50°C for 10 min.

Then, both the reaction mixtures were treated in the same manner as in Example 1 to obtain materials according to the present invention. These materials were named respectively Shaped materials A and B. That is, shaped material A was derived from the cross-linked de-N-acetylated glycolchitin and Shaped material B was derived from the cross-linked de-N-acetylated ethylchitin. The infrared absorption data and the results of colour reaction of the soluble parts and insoluble parts of the two products after treatment with an aqueous hydrochloric acid in the same manner as in Example 2 are shown in Table 2. From Table 2, the spherical products were confirmed to have been N-acylated on their surface.

TABLE 2
Properties of $HCl_{aq}$-treated substances

| Specimen | Infrared absorption ($cm^{-1}$) 1500 to 1530 | | Infrared absorption ($cm^{-1}$) 1550 | | Colour reaction | |
|---|---|---|---|---|---|---|
| | Insol[2] | Sol[1] | Insol | Sol | Insol | Sol |
| Shaped material A | No[3] | Yes[4] | Yes | No | No | Yes |
| Shaped material B | No | Yes | Yes | No | No | Yes |

Notes: 1) Sol. means the part of the substance dissolved in the aqueous hydrochloric acid, after recovery from the hydrochloric acid solution.
2) Insol. means the part of the substance not dissolved in the aqueous hydrochloric acid.
3) No means no absorption peak is present.
4) Yes means an absorption peak is present.

Example 4

$10^{-2}$ Kg (ten grams) of a starting material, spherical particles of carboxyethylchitin of a degree of cross-linking by epichlorohydrin of 0.1 and a degree of carboxyethylation of 0.5, were swollen with water. After washing the particles with a 1:1 by volume mixture of ethanol and toluene repeatedly to substitute the water by the solvent mixture, the solvent mixture in the particles was substituted for toluene only. The particles including the toluene were dispersed in $10^{-3}$m (one liter) of an aqueous 2N hydrochloric acid solution under agitation and treated for 15 min at 50°C. After separating the particles by filtration, the particles were put into $10^{-4}m^3$ (100 ml) of an aqueous methanol solution containing 10% by weight of sodium hydroxide (the volume ratio of water/methanol being 1:2). After separating the particles by filtration, the particles were washed with distilled water. The thus treated particles and starting material were respectively added to distilled water in which $10^{-2}$ kg (10 g) of the starting material of Example 3, that is spherical particles of glycol-chitin, had been dispersed. The particles of the starting material of Example 4 agglomerated whereas the spherical particles of the treated product of Example 4 were not agglomerated. It was confirmed that the latter had no ionic properties on its particles' surface.

Example 5

A glass column of $10^{-2}$m (10 mm) and $2 \times 10^{-1}$m (200 mm) in diameter and length, respectively, was filled with the spherical particles obtained in Example 1. Separately, after dispersing the starting material of Example 1 in an aqueous 2% by weight sodium chloride solution, a column with the same dimensions was filled with particles of the starting material of Example 1 and distilled water was passed through the filled column until no more sodium chloride was detected in the effluent to obtain a reference column.

$10^{-6}$ Kg (one milligram) of each of bovine serum fibrinogen (Fraction 1) and bovine serum albumin were dissolved into $10^{-5}m^3$ (10 ml) of distilled water respectively. The two solutions were mixed well to make a combined solution.

After pouring $10^{-6}m^3$ (one milliliter) of the combined solution into each of the two columns, the columns were developed with distilled water at a rate of $1.66 \times 10^{-8}m^3$/sec (one milliliter/min).

In the first 4 to 5 ml of effluent from the column filled with the spherical particles according to the present invention a substance having absorption in the ultraviolet region was detected. Such a substance was not detected in the effluent from the column filled with the starting material of Example 1. The substance having absorption in the ultraviolet region was identified by liquid-chromatography, etc. as fibrinogen.

The same experiments were repeated, however, using an aqueous 0.1N sodium chloride solution instead of distilled water to develop the column. In this case, in the first 10 to 12 ml of the effluent from the column filled with the spherical particles according to the present invention, albumin was detected, as a substance showing an ultraviolet absorption, without being accompanied by anything. In the effluent from the column filled with the starting material in Example 1, a compound showing ultraviolet absorption was detected in each of the first 5 to 7 ml and 10 to 12 fractions respectively. The compounds were identified as fibrinogen and albumin, respectively.

As is seen above, the shaped material according to the present invention shows an exclusive limit depending upon the molecular weight of protein. Accordingly, the shaped material according to the present invention can be used to fractionate proteins depending on their molecular weight.

Example 6

This example shows the results of measurement of mechanical strength of the shaped material according to the present invention, prepared in Example 1.

Measurement was carried out as follows:

After filling a column of $2 \times 10^{-2}$m (20 mm) and $1.25 \times 10^{-1}$m (125 mm) in diameter and height, respectively, to a height of $10^{-1}$m (100 mm) with the shaped material of the present invention distilled water was introduced from an inlet to the column via a pump fitted with a pressure gauge to examine the relationship between the introductory pressure and the rate of effluent of distilled water at the outlet of the column. A comparative measurement was carried out by using a de-acetylated product of not cross-linked carboxymethylchitin as the filling material for the column. This de-acetylated product was prepared as follows:

An aqueous 2% by weight solution of an acetate of the deacetylated product of carboxymethylchitin of a degree of carboxymethylation of 0.7 and a degree of deacetylation of 0.9 was added dropwise to a 10:1 by volume mixture of acetic anhydride and acetic acid. Reaction occurred under agitation for 5 min at room temperature. After the reaction was over, the reaction mixture was neutralized and the desired product was separated by filtration and washed with water.

The results of the mechanical strength test are shown in the Figure of the accompanying drawing. As is shown in the drawing, in the case of the comparative specimen, A, the deformation of the shaped material caused by the increased pressure reduced the rate of flow of distilled water. In the case of the shaped material according to the present invention, B, no reduction of the flow rate due to the increased pressure was observed. This demonstrates the superior mechanical strength of the shaped material according to the present invention.

**Claims**

1. A material produced by acylating or treating with an acid a surface of a material composed of a cross-linked ionic chitin derivative comprising a structural unit represented by the formula:

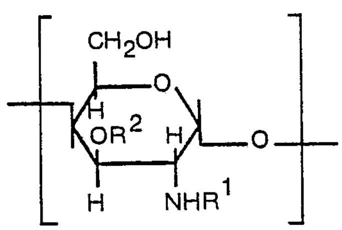

or

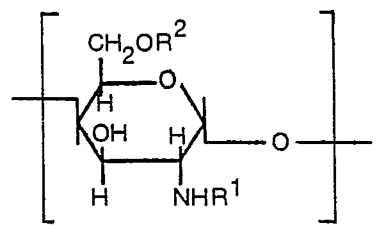

wherein

$R^1$ denotes a hydrogen atom, a group of formula $—C_nH_{2n+1}$, a group of formula $—(CH_2)_n\ OH$ or a group of formula $—CO(CH_2)_{n-1}CH_3$ and $R^2$ denotes a hydrogen atom, a group of formula $—C_{n'}H_{2n'+1}$, a group of formula $—(CH_2)_{n'}OH$, a group of formula

$$—(CH_2)_{n'}—\underset{\displaystyle OH}{\underset{|}{C}}H.CH_2OH$$

or a group of formula $—(CH_2)_{n'}COOM$, wherein *n* is an integer of 1 to 20, *n'* is an integer of 1 to 3, and M is a hydrogen atom, an alkali metal or an atomic equivalent amount of an alkaline earth metal.

2. A material according to claim 1 wherein the cross-linked ionic chitin derivative has a degree of cross-linking of from 0.01 to 0.3 per pyranose unit.

3. A material according to claim 1 or 2 wherein the cross-linked ionic chitin derivative has a degree of $R^2$ substitution of from 0.1 to 0.9.

4. A material according to claim 3 wherein the cross-linked ionic chitin derivative has a degree of carboxyalkylation of from 0.3 to 0.9.

5. A material according to any one of the preceding claims wherein the cross-linked ionic chitin derivative comprises structural units as defined in claim 1 wherein $R^1$ is H or $COCH_3$ such that said derivative has a degree of de-N-acetylation of from 0.1 to 1.0.

6. A material according to any one of the preceding claims wherein the acylation or treatment with acid has caused the formation of one or more of a chitin, N-acylated carboxyalkylchitosan of formula (VII): $-\!\!\left[C_6H_8O_3(OCH_2COOH)NHCOR\right]\!\!-$ wherein R represents $—(CH_2)_mCH_3$ and *m* is 0 or an integer from 1 to 20, N-acylated chitosan and N-, O-diacylated chitosan on at least the surface of the material.

7. A process for the preparation of a material as claimed in any one of the preceding claims, which process comprises:

(i) cross-linking natural chitin or regenerated chitin with a cross-linking agent,

(ii) reacting the cross-linked chitin with an etherifying agent to introduce the $OR^2$ group at the 3- or 6- position of the pyranose ring of structural units of the cross-linked chitin wherein $R^2$ is as defined in claim 1,

(iii) de-acetylating the cross-linked etherified chitin, and

(iv) acylating or treating with acid the surface of the de-acetylated, cross-linked, etherified chitin.

8. Use of a material as claimed in any one of claims 1 to 6 or which has been produced by a process as claimed in claim 7 in dialysis, as an ion-exchanger, in the purification and separation of glycoproteins, as an adsorbent for internal administration to adsorb and remove gastrointestinal toxins or as a covering material for adsorbents.

**Revendications**

1. Produit préparé par acylation ou traitement avec un acide d'une surface d'une matière composée d'un dérivé de chitine réticulé ionique comprenant un motif représenté par la formule:

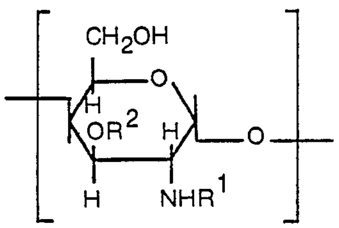

ou

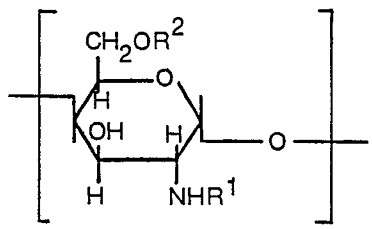

dans laquelle $R^1$ représente un atome d'hydrogène, un groupe de formule $—C_nH_{2n+1}$, un group de formule $—(CH_2)_nOH$ ou un groupe de formule $—CO(CH_2)_{n-1}CH_3$, et $R^2$ représente un atome d'hydrogène, un groupe de formule $—C_{n'}H_{2n'+1}$, un groupe de formule $—(CH_2)_{n'}OH$, un groupe de formule

$$—CH_2)_{n'}—\underset{\underset{OH}{|}}{CH}.CH_2OH$$

ou un groupe de formule $—(CH_2)_{n'}COOM$, dans laquelle n est un nombre entier de 1 à 20, n' est un nombre entier de 1 à 3 et M est un atome d'hydrogène, un métal alcalin ou une quantité atomique équivalente d'un métal alcalino-terreux.

2. Produit selon la revendication 1, dans lequel le dérivé de chitine réticulé ionique a un degré de réticulation de 0,01 à 0,3 par motif de pyrannose.

3. Produit selon la revendication 1 ou 2, dans lequel le dérivé de chitine réticulé ionique a un degré de substitution par $R^2$ de 0,1 à 0,9.

4. Produit selon la revendication 3, dans lequel le dérivé de chitine réticulé ionique a un degré de carboxy-alcoylation de 0,3 à 0,9.

5. Produit selon l'une quelconque des revendications précédentes, dans lequel le dérivé de chitine réticulé ionique comprend des motifs comme défini dans la revendication 1, où $R^1$ est H ou $COCH_3$, si bien que ledit dérivé a un degré de dé-N-acétylation de 0,1 à 1,0.

6. Produit selon l'une quelconque des revendications précédentes, dans lequel l'acylation ou le traitement avec un acide a provoqué la formation d'un ou plusieurs carboxyalcoylchitosanes N-acylés de chitine de formule (VII), $\text{—}[C_6H_8O_3(OCH_2COOH)NHCOR]\text{—}$, dans laquelle R représente $—(CH_2)_mCH_3$ et m est 0 ou un entier de 1 à 20, un chitosane N-acylé et un chitosane N-, O-diacylé sur au moins la surface de la matière.

7. Procédé pour la préparation d'un produit selon l'une quelconque des revendications précédentes, ce procédé comprenant:

1° la réticulation de la chitine naturelle ou de la chitine régénérée avec un agent réticulant;

2° la réaction de la chitine réticulée avec un agent éthérifiant pour introduire le groupe $OR^2$ sur la position 3 ou 6 du cycle pyrannose des motifs de la chitine réticulée dans laquelle $R^2$ est comme défini dans la revendication 1;

3° la désacétylation de la chitine réticulée éthérifiée, et

4° l'acylation ou le traitement avec un acide de la surface de la chitine réticulée, éthérifiée, désacétylée.

8. Utilisation d'un produit selon l'une quelconque des revendications 1 à 6, ou qui a été obtenu selon un procédé comme revendiqué dans la revendication 7 dans la dialyse, comme échangeur d'ions, dans la purification et la séparation des glycoprotéines, comme adsorbant pour l'administration interne pour adsorber et éliminer les toxines gastro-intestinales ou comme matière de recouvrement d'adsorbants.

## Patentansprüche

1. Ein Material, hergestellt durch Acylieren oder Behandlung mit einer Säure einer Oberfläche eines Materials, das aus einem vernetzten ionischen Chitin-Derivat zusammengesetzt ist, das eine Struktureinheit umfaßt, die durch eine der folgenden Formeln wiedergegeben wird:

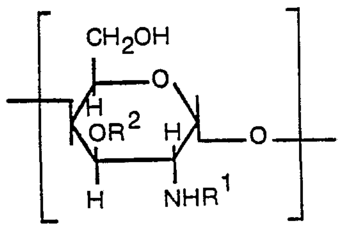

oder

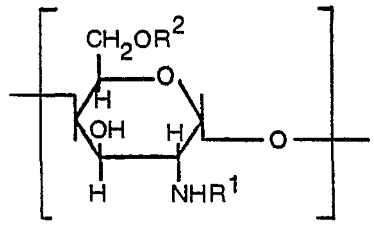

in denen $R^1$ ein Wasserstoffatom, eine Gruppe der Formel $—C_nH_{2n+1}$, eine Gruppe der Formel $—(CH_2)_nOH$ oder eine Gruppe der Formel $—CO(CH_2)_{n-1}CH_3$ bezeichnet, und $R^2$ ein Wasserstoffatom, eine Gruppe der Formel $—C_{n'}H_{2n'+1}$, eine Gruppe der Formel $—(CH_2)_{n'}OH$, eine Gruppe der Formel

$$—(CH_2)_{n'}—\underset{\displaystyle OH}{\underset{|}{CH}}—CH_2OH$$

oder eine Gruppe der Formel $—(CH_2)_{n'}COOM$ bezeichnet, worin n eine ganze Zahl von 1 bis 20 ist und n' eine ganze Zahl von 1 bis 3 ist und M eine Wasserstoffatom, ein Alkalimetall oder eine äquivalente Atommenge eines Erdalkalimetalls ist.

2. Material nach Anspruch 1, bei dem das vernetzte ionische Chitin-Derivat einen Vernetzungsgrad von 0,01 bis 0,3 pro Pyranose-Einheit aufweist.

3. Ein Material nach Anspruch 1 oder 2, bei dem das vernetzte ionische Chitin-Derivat einen $R^2$-Substitutionsgrad von 0,1 bis 0,9 aufweist.

4. Ein Material nach Anspruch 3, bei dem das vernetzte ionische Chitin-Derivat einen Carboxyalkylierungs-Grad von 0,3 bis 0,9 aufweist.

5. Material nach einem beliebigen der vorangehenden Ansprüche, bei dem das vernetzte ionische Chitin-Derivat in Anspruch 1 definierte Struktureinheiten umfaßt, in denen $R^1$ H oder $COCH_3$ ist, so daß dieses Derivat einen N-Deacetylierungsgrad von 0,1 bis 1,0 aufweist.

6. Ein Material nach einem beliebigen der vorangehenden Ansprüche, bei dem die Acylierung oder die Behandlung mit Säure zur Bildung eines oder mehrerer der Materialien Chitin, N-acyliertes Carboxyalkylchitosan der Formel (VII): $-[C_6H_8O_3(OCH_2COOH)NHCOR]-$, in der R $—(CH_2)_mCH_3$ bedeutet und m 0 oder eine ganze Zahl von 1 bis 20 ist, N-acyliertes Chitosan und N-, O-diacyliertes Chitosan wenigstens auf der Oberfläche des Materials führt.

7. Verfahren zur Herstellung eines Materials nach einem beliebigen der vorangehenden Ansprüche, wobei dieses Verfahren umfaßt:

(i) Quervernetzen von natürlichem Chitin oder von regeneriertem Chitin mit einem Vernetzungsmittel,

(ii) Umsetzen des vernetzten Chitins mit einem Veretherungsmittel zur Einführung der $OR^2$-Gruppe in der 3- oder 6- Stellung des Pyranoserings der Struktureinheiten des vernetzten Chitins, wobei $R^2$ wie in Anspruch 1 definiert ist,

(iii) Entacetylierung des vernetzten veretherten Chitins, und

(iv) Acylierung oder Behandlung der Oberfläche des entacetylierten, vernetzten, veretherten Chitins mit einer Säure.

8. Verwendung eines Materials nach einem beliebigen der Ansprüche 1 bis 6 oder des nach einem Verfahren nach Anspruch 7 erzeugten Produkts bei der Dialyse, als ein Ionenaustauscher, bei der Reinigung und Trennung von Glycoproteinen, als Adsorbens zur innerlichen Verabreichung zur Adsorption und Entfernung gastrointestinaler Toxine oder als Überzugsmaterial für Adsorbentien.

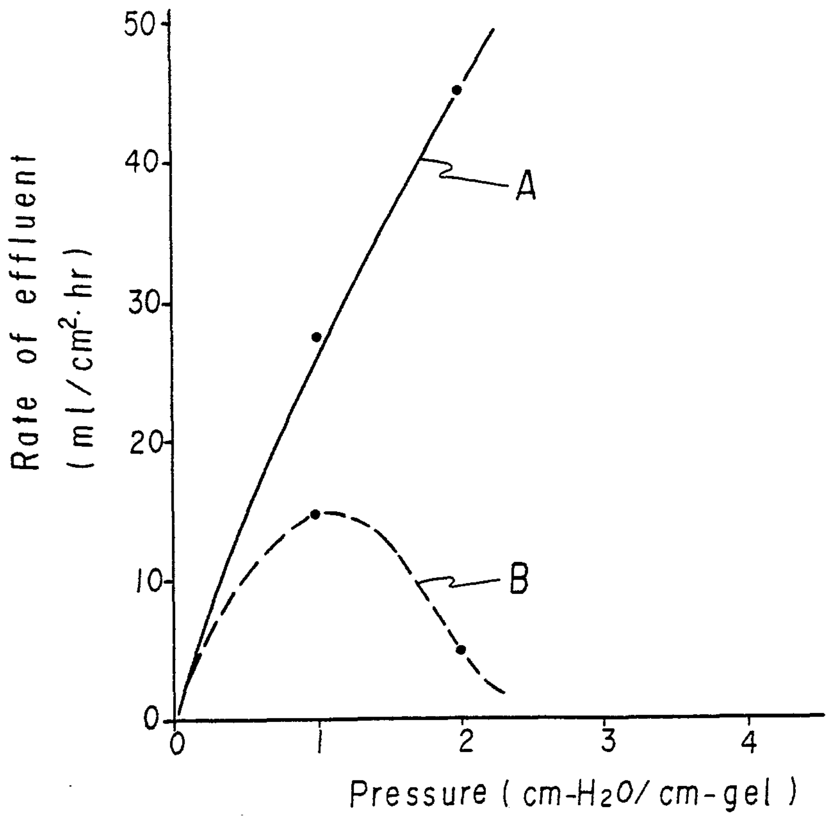

50
40
30
20
10
0
Rate of effluent
( ml / cm$^2$ · hr )
A
B
0
1
2
3
4
Pressure ( cm-$H_2O$/ cm-gel )